# EUROPÄISCHE PATENTSCHRIFT

(11) **EP 1 041 037 B1**
(45) Veröffentlichungstag und Bekanntmachung des Hinweises auf die Patenterteilung: **17.01.2007**
(21) Anmeldenummer: 00105993.0
(22) Anmeldetag: 27.03.2000
(51) Int. Cl.: C01B 3/36, C07C 11/24, C07C 2/78

(54) **Verfahren zur Herstellung von Acetylen und Synthesegas**
Process for the production of acetylene and synthesis gas
Procédé de production d'acétylène et de gaz de synthèse

(30) Priorität: 29.03.1999 DE 19914226
(43) Veröffentlichungstag der Anmeldung: 04.10.2000
(73) Patentinhaber: BASF Aktiengesellschaft, 67056 Ludwigshafen (DE)
(72) Erfinder: Stapf, Dieter, Dr., 68199 Mannheim (DE); Pässler, Peter, Dr., 67069 Ludwigshafen (DE); Bachtler, Michael, Dr., 67435 Neustadt (DE); Scheidsteger, Olaf, Dr., 68163 Mannheim (DE); Bartenbach, Bernd, Dr., 67117 Limburgerhof (DE)
(74) Vertreter: Isenbruck, Günter

(56) Entgegenhaltungen:
- EP-A- 0 287 380
- US-A- 2 679 543
- US-A- 2 697 032
- US-A- 2 764 554
- US-A- 2 884 472

## Beschreibung

Die Erfindung betrifft ein Verfahren zur Herstellung von Acetylen und Synthesegas.

Zahlreiche Verfahren zur unkatalysierten Herstellung von Acetylen basieren auf der Pyrolyse oder der partiellen Oxidation von Kohlenwasserstoffen. Als Einsatzstoffe kommen dabei kurzkettige Spezies, im C-Bereich von Methan, bis hin zu langkettigen Verbindungen des Rohöls in Frage. Eine Prozeßentwicklung, das Tauchflammenverfahren, ermöglicht es, zusätzlich hoch siedende Fraktionen, wie Rückstandsöle, einzusetzen. Prinzipiell haben bei pyrolytischen oder oxidativen Herstellungsverfahren von Acetylen thermodynamische und kinetische Parameter einen entscheidenden Einfluß auf die Wahl der Reaktionsbedingungen. Wichtige Voraussetzungen entsprechender Prozesse sind eine schnelle Energiezufuhr auf hohem Temperaturniveau - die maximale Reaktionstemperatur muß mehr als 1400°C betragen - äußerst kurze Verweilzeiten der Einsatzstoffe bzw. Reaktionsprodukte von 10⁻² bis 10⁻³ Sekunden, niedriger Partialdruck des Acetylens und schnelles Abschrecken der entstandenen Gase. Acetylen fällt bei der Pyrolyse und bei der partiellen Oxidation in einem Gasgemisch, dem sogenannten Spaltgas an. Dabei enthält das Spaltgas normalerweise etwa 5 bis 20 Vol.-% Acetylen. Letzteres wird durch selektive Lösungsmittel, wie N-Methylpyrrolidon, Dimethylformamid, Kerosin, Methanol oder Aceton, aus dem Spaltgas extrahiert und in weiteren Stufen gereinigt.

Die einzelnen Verfahren, bei denen Acetylen hergestellt wird, unterscheiden sich insbesondere hinsichtlich der Erzeugung der hohen Reaktionstemperaturen. Dabei spielen die Bereitstellung sowie die Übertragung der Wärmeenergie die entscheidende Rolle. Vom Prinzip her lassen sich zwei Verfahrensweisen unterscheiden:
- A: allotherme Pyrolyseverfahren mit zumeist elektrischer Aufheizung
- B: autotherme Verfahren, bei denen die Wärme aus der Teilverbrennung des Einsatzproduktes zur oxidativen Pyrolyse genutzt wird

### Zu A:

Hierzu gehört das Lichtbogenverfahren. Nach diesem Verfahren werden Kohlenwasserstoffe bis zu einem Siedepunkt von 200°C mit Hilfe eines ca. 1 m langen stabilisierten Lichtbogens pyrolysiert, der in seinem Zentrum eine Temperatur von bis zu 20.000°C aufweist. Am Ende des "Brenners" hat das Gasgemisch bei ca. 1,2 bar Betriebsdruck eine Temperatur von ca. 1800°C, die durch Einspritzen von Wasser schnell auf ca. 100°C abgesenkt wird. Die Verweilzeit in der Brennerzone beträgt einige Millisekunden, die Ausbeuten an Acetylen bzw. Ethylen (bei Ethylen in der Regel nur bei Vorquench mit Kohlenwasserstoffen) erreichen 1,0 bzw. 0,42 t pro 1,8 t eingesetzten Kohlenwasserstoff. Ein anderes Lichtbogenverfahren wurde in zwei großtechnischen Versuchsanlagen erprobt. H₂ als Wärmeträger wird zunächst in einem Lichtbogen auf 3000 bis 4000°C erhitzt und dabei zu 30 bis 65 % zu Atomen dissoziiert. Im anschließenden Reaktor können dann alle Arten von Kohlenwasserstoffen von Methan bis hin zum Rohöl in das Plasma eingedüst und gespalten werden. Das Spaltgas wird schnell abgeschreckt und getrennt. Beim Einsatz von Leichtbenzin können Acetylen-Ethylen-Ausbeuten von etwa 80 Gew.-% erhalten werden, wenn Nebenprodukte in den Spaltprozeß zurückgeführt werden. Die Acetylen-Konzentration im Spaltgas erreicht fast 14 Vol.-%.

### Zu B:

Ein dazu entwickeltes autothermes Spaltverfahren ist für Einsatzprodukte wie Methan, Flüssiggas oder Leichtbenzin geeignet. Die überwiegende Zahl der weltweit gebauten Anlagen basiert auf Erdgas als Einsatzprodukt; nur wenige verwenden Naphta als Rohstoff. Im technischen Prozeß werden beispielsweise Methan und Sauerstoff getrennt auf 500 bis 600°C vorgeheizt, gemischt und in einem speziellen Brenner unter Flammenbildung zur Reaktion gebracht. Das Verhältnis O₂/CH₄ wird mit etwa 1:2 so eingestellt, daß nur eine unvollständige Verbrennung erfolgen kann. In der Flamme findet sowohl die exotherme Oxidation eines Teils des CH₄ als auch die endotherme Dehydrodimerisierung des CH₄ in Acetylen und Wasserstoff statt. Nach einer Verweilzeit von wenigen Millisekunden schreckt man das Reaktionsgas durch Einspritzen von Wasser oder Quenchöl ab, da sonst Acetylen zu Ruß und Wasserstoff zerfallen würde. Die Bildung von Ruß kann jedoch nicht vollständig verhindert werden - pro 100 kg Acetylen fallen etwa 5 kg Ruß an. Die Abtrennung des Acetylens wird üblicherweise mit einem Extraktionsmittel, wie N-Methylpyrrolidon oder Dimethylformamid, vorgenommen. Fraktionierte Desorption und geeignete Rektifikationsstufen dienen dann zur Abtrennung von mitgelösten Begleitkomponenten. Im Spaltgas beträgt der Volumenanteil des Acetylens etwa 8 %. Die Hauptkomponenten sind Wasserstoff mit 57 Vol.% und Kohlenmonoxid mit 26 Vol. %. In diesem Verhältnis stellen die Hauptkomponenten ein gut einsetzbares Synthesegas dar. Die autotherme Herstellung von Acetylen ist stets mit der Herstellung von Synthesegas verbunden.

Im folgenden sollen einige verwendete Begriffe definiert werden:

Mit Aufheizen sollen alle die Maßnahmen und Prozesse verstanden werden, die zu einer Temperaturerhöhung führen. Ein Medium, z.B. ein Ausgangsgemisch zur Herstellung von Acetylen und Synthesegas, kann beispielsweise durch Zünden (dadurch wird eine exotherme Reaktion ausgelöst), durch die Zufuhr von Energie (z.B. von außen) oder durch exotherme Reaktionen bei gleichzeitiger oder vorausgehender Zufuhr von Energie (z.B. durch Vorheizen), aufgeheizt werden. Unter Ausgangsgemisch wird das Gemisch verstanden, das für den Prozeß der Acetylen/Synthesegas-Herstellung eingesetzt wird. Dieses kann prinzipiell variieren, und es enthält je nach gewünschtem Synthesegas entsprechend verschiedene Ausgangsstoffe. Es ist in dem Ausgangsgemisch stets molekularer Sauerstoff und/oder eine oder mehrere das Element Sauerstoff enthaltende Verbindung enthalten. Molekularer Sauerstoff kann dem Ausgangsgemisch in Form von Luft, Luft/Sauerstoff-Mischungen oder reinem Sauerstoff bereitgestellt werden. Die das Element Sauerstoff enthaltenden Verbindungen können als Wasserdampf und/oder Kohlendioxid bereitgestellt werden. Außerdem enthält das Ausgangsgemisch einen oder mehrere Kohlenwasserstoffe. Dabei enthält das Ausgangsgemisch häufig, insbesondere dann wenn Methanol-Synthesegas hergestellt werden soll, zu einem großen Teil Erdgas, aber auch zum Beispiel Flüssiggas wie Propan oder Butan, Leichtbenzin wie Pentan oder Hexan, Benzol oder andere Aromaten, Pyrolysebenzin oder Destillationsrückstände aus der Erdölraffinerie. Die Umwandlung des Ausgangsgemischs in ein Acetylen/Synthesegas enthaltendes Gemisch wird als thermische Behandlung bezeichnet. Die zugrundeliegenden Reaktionstypen sind vorwiegend Verbrennung (Totaloxidation), partielle Verbrennung (partielle Oxidation bzw. oxidative Pyrolyse) und Pyrolysereaktionen (Reaktionen ohne die Beteiligung von Sauerstoff). Unter indirekter Kühlung wird das Abkühlen des Reaktionsgemischs verstanden, wenn das eingesetzte Kühlmittel dabei nicht in direkten Kontakt mit dem Reaktionsgemisch gelangt. Umgekehrt tritt beim direkten Quench das Kühlmittel direkt in Kontakt mit dem Reaktionsgemisch.

Den bekannten Acetylen-Herstellungsmethoden ist gemeinsam, daß die Reaktionstemperatur über 1400°C liegt und daß die Verweilzeiten im Millisekundenbereich liegen. Zur Vermeidung von Folgereaktionen (beispielsweise Rußbildung) muß das Reaktionsgas dann schnell durch direkten Quench abgeschreckt werden, wobei ein Quenchmittel direkt eingespritzt wird. Dabei kühlt sich das entsprechende Gemisch je nach eingesetztem Quenchmittel verschieden stark ab - bei Öl als Quenchmittel auf ca. 300°C und bei Wasser als Quenchmittel auf ca. 100°C. Aus dem resultierenden Gemisch wird das Acetylen durch selektive Lösungsmittel ausgewaschen.

Vorstehendes Prinzip der Acetylen/Synthesegas-Herstellung ist in der DE-A-44 22 815 offenbart. Dabei wird das Ausgangsgemisch im Anschluß an die getrennte Vorwärmung in einer Mischkammer erzeugt. Die Reaktion findet anschließend an einem Brennerblock in einem Brennraum statt. Der Abbruch der Reaktion erfolgt in einem Quenchbehälter. Da Acetylen bei hohen Temperaturen thermodynamisch instabil ist und zum schnellen Zerfall neigt, muß das acetylenhaltige Produktgemisch schlagartig abgekühlt werden (durch direkten Quench) - eine indirekte Kühlung wäre dabei zu träge.

Das bisherige Prinzip der Acetylen-Synthesegasherstellung, wie in der DE-A-44 22 815 beschrieben, ist mit erheblichen Nachteilen behaftet. Ein wesentlicher Nachteil ist es, daß durch das Quenchen (direkter Quench) eine optimale Rückgewinnung der Energie unmöglich wird. Die entsprechende Quenchflüssigkeit weist nach ihrem Einsatz typischerweise Temperaturen von höchstens 300°C auf, d.h. die Energie wird auf relativ niedrigem Temperaturniveau (ca. 200 bis 300°C) zurückgewonnen, obwohl sie bei hohen Temperaturniveau (ca. 1500 bis 1600°C) anfällt. Der Einsatzbereich einer solchen, relativ kühlen Flüssigkeit zu Heizzwecken ist stark eingeschränkt. Außerdem ist die Quenchflüssigkeit in der Regel stark mit Rußkoks und aromatische Komponenten belastet, so daß eine weitere Verwendung erschwert wird. Der hohe energetische Aufwand, der für die Erzeugung der höher als 1400°C liegenden Reaktionstemperatur notwendig ist, ist ein weiterer Nachteil. Ein weiteres Problem beim bisherigen Verfahren ergibt sich aus der starken Rußbildung, die bei den hohen Prozeßtemperaturen besonders stark ist. Die Rußbildung macht sich nicht nur dadurch negativ bemerkbar, daß die Ausbeute an Synthesegas und Acetylen reduziert wird, sondern auch durch die Verschmutzungsproblematik der eingesetzten Apparaturen. Aufwendig ist auch die Reinigung der u.a. durch Ruß bzw. Rußkoks verschmutzten Quenchflüssigkeit und des Spaltgasgemisches - Reinigungs- bzw. Trennverfahren müssen daher meist nachgeschaltet werden.

US 2,679,543 betrifft ein Verfahren zur Herstellung von Acetylen, in dem ein Ausgangsgemisch aus einem Kohlenwasserstoff und Sauerstoff vorgeheizt wird und bei einer Reaktionstemperatur von 1100 bis 1500 °C zu Acetylen reagiert. Das heiße Reaktionsgemisch wird durch direktes Quenchen auf 500 bis 600 °C abgekühlt, anschließend wird durch indirekte Kühlung gekühlt.

Der Erfindung liegt die Aufgabe zugrunde, den Energiebedarf der Acetylen/Synthesegas-Herstellung zu reduzieren und außerdem die Möglichkeit einer effektiveren Rückgewinnung der eingesetzten Energie zu schaffen. Außerdem soll die bei dem Verfahren entstehende Rußmenge gering gehalten werden.

Die Lösung dieser Aufgabe geht aus von dem Verfahren zur Herstellung von Acetylen und Synthesegas durch thermische Behandlung eines Ausgangsgemisches, das einen oder mehrere Kohlenwasserstoffe und außerdem molekularen Sauerstoff und/oder ein oder mehrere das Element Sauerstoff aufweisende Verbindungen enthält, wobei das Ausgangsgemisch höchstens bis auf maximal 1400 °C aufgeheizt wird, in einem Reaktor zur Reaktion gebracht und anschließend durch kombinierte Anwendung von indirekter Kühlung und direktem Quench abgekühlt wird. Das erfindungsgemäße Verfahren ist dadurch gekennzeichnet, daß durch direkten Quench höchstens auf 1000 °C abgekühlt wird.

Wie vorstehend bereits erläutert, sollen unter Aufheizen alle Maßnahmen und Prozesse verstanden werden, die zu einer Temperaturerhöhung des Ausgangsgemischs führen. Das Ausgangsgemisch zur Herstellung von Acetylen und Synthesegas kann beispielsweise durch Zünden - dadurch wird eine exotherme Reaktion ausgelöst - durch die Zufuhr von Energie (z.B. durch Heizen von außen) oder durch exotherme Reaktionen bei gleichzeitiger oder vorausgehender Zufuhr von Energie (z.B. durch Vorheizen), aufgeheizt werden. Das Ausgangsgemisch bzw. aus dem Ausgangsgemisch entstehende Reaktions-mischungen weisen demnach erfindungsgemäß während des Verfahrens Temperaturen von höchstens maximal 1400°C auf. In einer bevorzugten Ausführungsform wird das Ausgangsgemisch ausschließlich durch die in dem Reaktor frei werdende Reaktionswärme aufgeheizt.

Im Gegensatz zu bekannten Verfahren ist die mittlere Verweilzeit in dem Reaktor vergleichsweise lang - sie beträgt in der Regel mindestens 10 ms.

Die thermische Behandlung findet in der Regel bei Temperaturen zwischen 1200°C und 1400°C statt. Besonders bevorzugt ist dabei der Bereich bis 1350°C. Dabei wird meist ausschließlich die in dem Reaktor freiwerdende Reaktionswärme genutzt, so daß auf eine elektrische oder thermische Vorheizung in der Regel weitestgehend verzichtet werden kann.

"Schnelles Quenchen" wird in der Literatur als Voraussetzung für das Erreichen von akzeptabelen Acetylen-Ausbeuten angesehen [Ullmann' s Encyclopedia of Industrial Chemistry, 5^{th} Rev. Ed., Vol. A1, Verlag Chemie, Weinheim, 1985, Seite 106]. Es soll aber nur deshalb schnell gequencht werden, da es als notwendig angesehen wird, die Reaktion bei hohen Temperaturen (Temperaturen höher als 1400°C) durchzuführen - müssen die Verweilzeiten bei den hohen Temperaturen kurz gehalten werden, muß entsprechend schnell abgekühlt werden. Daher ist nicht nur die Tatsache, daß die Acetylen/Synthesegas-Herstellung auch bei vergleichsweise langsamer indirekter Kühlung erfolgen kann, sondern auch die Tatsache, daß die Reaktion bei Temperaturen unterhalb von 1400°C durchgeführt werden kann, überraschend.

Der Vorteil, der sich aus der niedrigeren Prozeßtemperatur ergibt, ist einmal, daß die Rußbildung nur gering ist. Andererseits ist der Energiebedarf deutlich niedriger als bei den bisherigen Verfahren. Eine effektivere Rückgewinnung der Energie ist bei dem erfindungsgemäßen Verfahren möglich. Bei indirekter Kühlung kann durch den Einsatz eines geeigneten Wärmetauschers wertvoller Hochdruckdampf erzeugt werden (mit diesem kann man z.B. eine Turbine antreiben).

Molekularer Sauerstoff wird dem Ausgangsgemisch meist in Form von Luft oder Luft/Sauerstoff-Mischungen bereitgestellt. Das Element Sauerstoff enthaltende Verbindungen werden in der Regel als Wasserdampf und/oder als Kohlendioxid bereitgestellt. Meist wird dabei rezykliertes Kohlendioxid eingesetzt. Es wird dabei Kohlendioxid, das in dem abgekühlten Reaktionsgemisch enthalten ist, bereitgestellt, wobei zu diesem Zweck in der Regel das gesamte Kohlendioxid enthaltende Reaktionsgemisch rezykliert wird.

Wie vorstehend bereits erwähnt, ist die Zusammensetzung des Ausgangsgemisches variabel. Die Zusammensetzung des eingesetzten Ausgangsgemisches richtet sich in der Regel nach der Verwendung des herzustellenden Spaltgases. Wichtige Spaltgase, die mit dem erfindungsgemäßen Verfahren hergestellt werden können, sind beispielsweise Acetylen/Methanol-Synthesegas, Acetylen/Ammoniak-Synthesegas, Acetylen/Wasserstoff-Reichgas, Acetylen/-Kohlemonoxid-Reichgas, Acetylen/Oxogas, Acetylen/Ethylen-Synthesegas. Entsprechend dem herzustellenden Spaltgas muß die Zusammensetzung des Ausgangsgemischs gewählt werden. Neben Erdgas können Flüssiggas (Propan, Butan), Leichtbenzin, Aromaten, Pyrolysebenzin-Öl ( aus Crackprozessen) und/oder Vakuumdestillationsrückstände aus Erdölraffinerien verwendet werden. Ausgangsgemische können (bis zu etwa 10 Vol.-%) rückgeführtes Spaltgas, Recyclegase wie Rest-Methan aus der Spaltgasreinigung, Restgase aus anderen Prozessen oder Synthesegas beinhalten. Dies kann zum Beispiel dadurch erreicht werden, daß eine Teilmenge des abgekühlten Reaktionsgemischs rezykliert wird. Beim Einsatz höherer Kohlenwasserstoffe, insbesondere bei den bei Umgebungstemperatur flüssigen Kohlenwasserstoffen, kann sich eine Beimischung von bis zu 50 Vol.-%, bevorzugt bis zu 25 Vol.-% Wasserdampf zum Ausgangsgemisch günstig auf die Temperaturführung des Prozesses und auf den Wasserstoffanteil im Spaltgas auswirken. Sauerstoff kann dem Ausgangsgemisch in Form von Luft bereitgestellt werden. In diesem Fall erhält man Ammoniak-Synthesegas (ideale Zusammensetzung Wasserstoff zu Stickstoff 3:1) und Acetylen. Wasserdampfzugabe zu Erdgas mit hohem Methananteil führt bevorzugt zu Acetylen/Wasserstoff-Reichgas. Die Verwen-dung höherer Kohlenwasserstoffe führt zu Acetylen/Kohlenmonoxid-Reichgas.

Die Reaktion zur Herstellung von Acetylen/Synthesegas kann mit dem erfindungsgemäßen Verfahren bei beliebigem Druck, bevorzugt bei Atmosphärendruck durchgeführt werden. Als Reaktoren eignen sich bevorzugt Flammenreaktoren, regenerative Reaktoren, rekuperative Reaktoren oder Strömungsreaktoren, insbesondere Rohrreaktoren. Eingesetzte Flammenreaktoren weisen häufig einen Drallbrenner oder einen Brennerblock mit anschließendem Brennraum auf. In Frage kommende Flammenreaktoren arbeiten beispielsweise mit Vormisch-flamme oder Diffusionsflamme. Eingesetzte Strömungsreaktoren enthalten häufig eine Vormischflamme. Die Verweilzeit in dem Reaktor ist in der Regel kürzer als 1 s - jedoch die Abkühlphase in der Regel länger als diejenigen Abkühlphasen, die bei bekannten Verfahren realisiert werden. Der direkte Quench kann beispielsweise durch die Eindüsung von Quenchöl, Wasser, Dampf oder kalten Rückführgasen durchgeführt werden. Bei der Verwendung von Kohlenwasserstoffen als Quenchmittel können gleichzeitig Crackprozesse eingeleitet werden (Crackung der in dem Quenchmittel enthaltenen Kohlen-wasserstoffe). Im Prinzip kann zum Zweck der indirekten Kühlung jeder beliebige Wärmetauschertyp verwendet werden. Mit dem aufgeheizten Kühlmittel kann beispielsweise ein Hochdruckdampferzeuger oder ein Einsatzstoffvorwärmer betrieben werden.

Falls Methanol-Synthesegas erhalten werden soll, ist eine bevorzugte Zusammensetzung des Ausgangsgemischs: 60 bis 70 Vol.-% Erdgas (MethanAnteil: ca. 90 Vol.-%) und Sauerstoff als verbleibende Volumenprozente. Das optimale Methan: Sauerstoff-Verhältnis beträgt somit ca. 2:1.

Eine weitere bevorzugte Zusammensetzung des Ausgangsgemischs ist, falls Methanol-Synthesegas erhalten werden soll: 30 bis 50 Vol.-% molekularer Sauerstoff, 30 bis 50 Vol.-% Wasserdampf und als verbleibende Volumenprozente Kohlenwasserstoffe mit einem hohen C : H - Verhältnis (C : H - Verhältnis von mindestens 0,5 - z.B. Aromaten).

## Patentansprüche

1. Verfahren zur Herstellung von Acetylen und Synthesegas durch thermische Behandlung eines Ausgangsgemisches, das einen oder mehrere Kohlenwasserstoffe und außerdem molekularen Sauerstoff und/oder eine oder mehrere das Element Sauerstoff aufweisende Verbindungen enthält, wobei das Ausgangsgemisch höchstens bis auf maximal 1400 °C aufgeheizt wird, in einem Reaktor zur Reaktion gebracht und anschließenddurch kombinierte Anwendung von indirekte Kühlung und direktem Quench abgekühlt wird, **dadurch gekennzeichnet, daß** durch direkten Quench höchstens auf 1000 °C abgekühlt wird.

2. Verfahren nach Anspruch 1, **dadurch gekennzeichnet, daß** das Ausgangsgemisch ausschließlich durch die in dem Reaktor frei werdende Reaktionswärme aufgeheizt wird.

3. Verfahren nach Anspruch 1 oder 2, **dadurch gekennzeichnet, daß** die Reaktion bei beliebigem Druck, bevorzugt bei Atmosphärendruck, abläuft.

4. Verfahren nach einem der Ansprüche 1 bis 3, **dadurch gekennzeichnet, daß** der Reaktor als Flammenreaktor, als regenerativer Reaktor, als rekuperativer Reaktor oder als Strömungsreaktor, insbesondere als Rohrreaktor, ausgebildet ist.

5. Verfahren nach einem der Ansprüche 1 bis 4, **dadurch gekennzeichnet, daß** eine Teilmenge des abgekühlten Reaktionsgemischs rezykliert wird.

6. Verfahren nach einem der Ansprüche 1 bis 5, **dadurch gekennzeichnet, daß** molekularer Sauerstoff dem Ausgangsgemisch in Form von Luft oder Luft/Sauerstoff-Mischungen bereitgestellt wird.

7. Verfahren nach einem der Ansprüche 1 bis 6, **dadurch gekennzeichnet, daß** die das Element Sauerstoff enthaltenden Verbindungen als Wasserdampf und/oder Kohlendioxid bereitgestellt werden.

## Claims

1. A process for the preparation of acetylene and synthesis gas by thermal treatment of a starting mixture comprising one or more hydrocarbons and in addition molecular oxygen and/or one or more compounds comprising the element oxygen, in which the starting mixture is at most heated to a maximum of 1400°C, brought to reaction in a reactor and subsequently cooled by the combined use of indirect cooling and direct quenching, wherein direct quenching effects cooling to at most 1000°C.

2. The process according to claim 1, wherein the starting mixture is heated exclusively by the heat of reaction liberated in the reactor.

3. The process according to claim 1 or 2, wherein the reaction proceeds at any desired pressure, preferably at atmospheric pressure.

4. The process according to one of claims 1 to 3, wherein the reactor is in the form of a flame reactor, regenerative reactor, recuperative reactor or flow reactor, in particular tubular reactor.

5. The process according to one of claims 1 to 4, wherein some of the cooled reaction mixture is recycled.

6. The process according to one of claims 1 to 5, wherein molecular oxygen is provided to the starting mixture in the form of air or air/oxygen mixtures.

7. The process according to one of claims 1 to 6, wherein the compounds comprising the element oxygen are provided in the form of steam and/or carbon dioxide.

## Revendications

1. Procédé de préparation d'acétylène et de gaz de synthèse par traitement thermique d'un mélange initial contenant un ou plusieurs hydrocarbures et en outre de l'oxygène moléculaire et/ou un ou plusieurs composés présentant l'élément oxygène, le mélange initial étant chauffé au plus à un maximum de 1400°C, amené à réagir dans un réacteur et enfin refroidi par application combinée d'un refroidissement indirect et d'une trempe directe, **caractérisé en ce que** la trempe directe refroidit au plus à 1000°C.

2. Procédé selon la revendication 1, **caractérisé en ce que** le mélange initial est chauffé exclusivement par la chaleur réactionnelle ayant libre cours dans le réacteur.

3. Procédé selon la revendication 1 ou 2, **caractérisé en ce que** la réaction se déroule sous une pression quelconque, de manière préférée sous pression atmosphérique.

4. Procédé selon l'une quelconque des revendications 1 à 3, **caractérisé en ce que** le réacteur est conçu en tant que réacteur à flamme, en tant que réacteur à régénération, en tant que réacteur à récupération ou en tant que réacteur en écoulement, en particulier en tant que réacteur tubulaire.

5. Procédé selon l'une quelconque des revendications 1 à 4, **caractérisé en ce qu'**une quantité partielle du mélange réactionnel refroidi est recyclée.

6. Procédé selon l'une quelconque des revendications 1 à 5, **caractérisé en ce que** le mélange initial est approvisionné en oxygène moléculaire sous forme d'air ou de mélanges air/oxygène.

7. Procédé selon l'une quelconque des revendications 1 à 6, **caractérisé en ce que** les composés contenant l'élément oxygène sont mis à disposition sous forme de vapeur d'eau et/ou de dioxyde de carbone.
